# EUROPÄISCHE PATENTANMELDUNG

(11) **EP 1 051 947 A2**
(43) Veröffentlichungstag der Anmeldung: **15.11.2000**
(21) Anmeldenummer: 00109725.2
(22) Anmeldetag: 08.05.2000
(51) Int. Cl.: A61B 17/70

(54) **Schraube zur Anbringung eines Implantats zur Wirbelsäulenstabilisierung und Verlängerungsstab zum Positionieren und Eindrehen der Schraube**

(30) Priorität: 11.05.1999 DE 19921551
(71) Anmelder: Kluger, Patrick, Dr. med., D-89155 Erbach (DE)
(72) Erfinder: Buchholz, Eva-Maria, 51429 Bergisch-Gladbach (DE)
(74) Vertreter: Hentrich, Swen Dipl.-Phys. Dr.

(57) **Zusammenfassung**

Es wird eine Schraube zur Anbringung eines Implantats für die Wirbelsäulenstabiliesierung mit einem Gewindeabschnitt zum Einschrauben in einen Wirbelkörper und einen Schraubenkopf (3), in welchem eine Gewindebohrung angeordnet ist, vorgeschlagen, wobei der Schraubenkopf (3) kugelförmig ist und wobei der Oberflächenbereich des Schraubenkopfs (3) um die Gebindebohrungsöffnung ebenfalls kuglig gewölbt ist und eine Verzahnung aufweist. Des Weiteren wird ein Verlängerungsstab zum Positionieren und Eindrehen einer Schraube zur Anbringung eines Implantats für die Wirbelsäulenstabilisierung vorgeschlagen, welche sich am Kopf einer Schraube durch eine Schwenkbewegung verankern läßt. Bei einem weiteren Verlängerungsstab ist ein Klinkenorgan (24) vorhanden, das in eine Ausnehmung (14) einrastet, die am Kopf einer Schraube vorgesehen ist.

## Beschreibung

Die Erfindung betrifft eine Schraube zur Anbringung eines Implantats zur Wirbelsäulenstabilisierung mit einem Gewindeabschnitt zum Einschrauben in einen Wirbelkörper und einen Schraubenkopf, in welchem eine Gewindebohrung angeordnet ist sowie einen Verlängerungsstab zum Positionieren und Eindrehen einer Schraube zur Anbringung eines Implantats für die Wirbelsäulenstabilisierung.

### Stand der Technik:

Schrauben der einleitend bezeichneten Art sind bereits bekannt geworden. In der Europäischen Patentschrift EP 0 641 548 B1 ist beispielsweise eine Schraube für eine lumbosakrale Rückratstütze offenbart, die einen teilweise kugelförmigen Kopf aufweist, der eine verzahnte, zur Längsachse der Schraube um einen Winkel zwischen ungefähr 30 und 45 Grad geneigte Oberfläche und eine senkrecht zu dieser verzahnten Oberfläche angeordnete Gewindebohrung besitzt. Durch die geneigte Oberfläche wird insbesondere die Montage eines Längsträgers an den Schrauben erleichtert. Allerdings weisen derartige Schrauben den Nachteil auf, daß zum Einschrauben in einen entsprechend vorbereiten Wirbelkörper ein Verlägerungsstab notwendig ist, der den abgeschrägten Kopf der Schraube untergreift und dementsprechend das Einscbrauben nur bis zum Anschlag des Verlägerungsstabes an der Knochenoberfläche erlaubt.

Darüber hinaus ist die Fertigung von Schrauben mit abgeschrägtem Kopf vergleichsweise aufwendig.

### Aufgabe und Vorteil der Erfindung:

Der Erfindung liegt die Aufgabe zugrunde, eine Schraube der einleitend bezeichneten Art und einen Verlängerungsstab bereitzustellen, so daß insbesondere ein tieferes Einachrauben der Schraube in einen Wirbelkörper und eine günstigere Positionierung des an einer Schraube befestigten Implantats möglich wird.

Diese Aufgabe wird ausgebend von einer Schraube der einleitend bezeichneten Art dadurch gelöst, daß der Schraubenkopf kugelförmig ist, wobei der Oberflächenbereich des Schraubenkopfes um die Gewindebohrungsöffnung ebenfalls kugelig gewölbt ist und eine Verzahnung aufweist. Eine Schraube mit im Wesentlichen kugelförmigem Kopf läßt sich zunächst vergleichsweise einfacher herstellen, da nicht z. B. zusätzlich eine Schrägfläche wie bei einer einleitend beschriebenen Schraube, angebracht werden muß. Des Weiteren kann durch die kuglige Kopfform der Schraube ein Verlängerungsstab eines Manipulatorsystems zum Einachrauben der Schraube verwendet werden, der beim zug- und drehstabilen Anbringen am Schraubenkopf diesen nicht untergreifen muß. Damit bietet sich die Möglichkeit, die Schraube bis zum Kugelkopfansatz in einen Wirbelkörper einzusetzen, wodurch ein an den Schrauben befestigtes Implantat weniger aufträgt und außerdem die Belastbarkeit des Implantats erhöht wird, da sich die Hebel bis zur Knochenoberfläche verkürzen. Bei dem oben zitierten bekannten abgeschrägten Schraubenkopf hingegen läßt sich ein Verlängerungsstab eines Manipulatorsystems zum Eindrehen der Schraube aufgrund des durch die Abschrägung fehlenden Schraübenkopfmaterials nur anbringen, wenn ein Befestigungsstück eines Verlängerungsstabes den Schraubenkopf untergreift.

Im Weiteren bietet der kugelförmige Schraubenkopf die Möglichkeit, eine Gewindebohrung zur Befestigung eines Implantats, z. B. eines Längsträgers, der mehrere Schrauben verbindet, in nahezu jeder Neigung zur Schraubenlängsachse anzubringen. Denn anders als bei einem abgeschrägten Schraubenkopf muß bei einem kugelförmigen Schraubenkopf keine Materialschwächung berücksichtigt werden, wenn sich die Gewindeböhrung durch einen entsprechenden Neigungswinkel bis in den Bereich des Schraubenschaftes erstreckt. Im Weiteren weist bei einem kugelförmig ausgebildeten Schraubenkopf für den bevorzugten Fall einer senkrecht zur kugelig gewölbten Oberfläche des Schraubkopfes verlaufenden Längsachse der Gewindebohrung eine Gewindebohrung unabhängig vom Neigungswinkel immer eine gleiche ausreichend dimensionierte Länge auf. Dies erlaubt überdies Schrauben für die Gewindebohrung zu verwenden, die nicht vollständig den Schraubenkopf durchdringen müssen, wodurch bei einer durchgehenden Gewindebohrung die Öffnung für gegebenenfalls die Befestigung eines Verlängerungsstabes eines Manipulatorsystems genutzt werden kann. In einer vorteilhaften Weiterbildung der Erfindung ist die Längsachse der Gewindebohrung zur Längsachse der Schraube um einen Winkel von 20 bis 50 Grad geneigt.

Um einen Verlängerungsstab eines Manipulatorsystem in einfacher Weise am kugelförmigen Schraubenkopf verdrehsicher anbringen zu können, wird im Weiteren vorgeschlagen, daß der kugelförmige Schraubenkopf zwei im Wesentlichen parallel zur Längsachse der Schraube zur Gewindebohrung symmetrisch angeordnete abgeflachte Abschnitte aufweist. In diesem Zusammenhang ist es außerdem vorteilhaft, wenn im Bereich der abgeflachten Abschnitte eine vorzugsweise bogenförmige Nut vorgesehen ist, in welche sich Vorsprünge eines Verlängerungsstabes einführen lassen. Damit wird ein Verlängerungsstab in Längsrichtung der Schraube festgelegt.

In einer außerdem bevorzugten Ausführungsform der Erfindung weist der kugelförmige Schraubenkopf zur Anbringung eines Verlängerungsstabes auf der der Öffnung der Gewindebohrung entgegengesetzten Seite eine Ausnehmung auf, z. B. in Form einer im Wesentlichen quer zur Längsachse der Schraube verlaufenden Nut. In diese Ausnehmung bzw. Nut kann z. B. ein an einem Verlängerungsstab ungeordnetes Klinkenelement einrasten, um den Verlängerungsstab gegen Verkippen und/oder eine Bewegung in Längsrichtung der Schraube zu sichern.

Zur Befestigung eines Längsträgers, mit welchem sich mehrere Schrauben verbinden lassen, wird überdies vorgeschlagen, daß am Schraubenkopf eine Klemmschelle mittels eines Gewindebolzens angeordnet ist, der in die Gewindebohrung eingeschraubt wird. In diesem Zusammenhang ist es außerdem vorteilhaft, wenn der am kugelförmigen Schraubenkopf anliegende Schenkel der Klemmschelle dem kugelförmigen Kopf angeformt ist und eine Verzahnung aufweist, die zu der am Kopf angebrachten Verzahnung paßt.

Bin Verlängerungsstab zum Positionieren und Eindrehen einer Schraube zur Anbringung eines Implantats für die Wirbelsäulenstabilisierung ist vorzugsweise derart ausgestaltet, daß er sich am Kopf einer Schraube durch eine Schwenkbewegung verankern läßt. Dazu ist es überdies vorteilhaft, daß der Verlängerungsstab ein den Kopf der Schraube umgreifendes Schalenelement mit gegenüberliegenden Vorsprüngen aufweist, die in Führungsnuten, vorzugsweise bogenförmige Führungsnuten am Kopf einer Schraube einschwenkbar sind.

Im Weiteren ist es besonders günstig, wenn ein Stiftelement zur Blockierung der Schwenkbewegung am Verlängerungsstab vorgesehen ist.

Bei einem ebenfalls bevorzugten Verlängerungsstab ist ein Klinkenorgan vorgesehen, das in eine Ausnehmung am Kopf einer Schraube einrastbar ist. Auf diese Weise läßt sich z. B. eine Schwenkbewegung eines Verlängerungsstabs blockieren, jedoch ebenso eine Bewegung in Längsrichtung verhindern.

### Zeichnungen:

Mehrere Ausführungsbeispiele der Erfindung sind in den Zeichnungen dargestellt und in der nachfolgenden Beschreibung unter Angabe weiterer Vorteile mit Einzelheiten näher erläutert.

Es zeigen
- Figur 1a und b: eine Pedikelschraube in einer Seiten- und Vorderansicht,
- Figur 2: einen Gewindebolzen zum Eindrehen in die im Schraubenkopf einer Pedikelschraube angeordnete Gewindebohrung in Seitenansicht,
- Figur 3: eine Klemmschelle zur Befestigung eines Längsträgers für die Verbindung mehrerer Schrauben in Seitenansicht,
- Figur 4: in teilweise Schnittdarstellung die Fixierung einer Schraube nach Figur 1 a und b in einem Wirbelkörper.
- Figur 5 a und b: eine Pedikelschraube mit aufgesetztem Verlängerungsstab in einer Seiten- und Vorderansicht,
- Figur 6 a und b: eine Pedikelschraube mit einer zweiten Ausführungsform eines aufgesetzten Verlängerungsstabes in einer Seiten- sowie Vorderansicht und

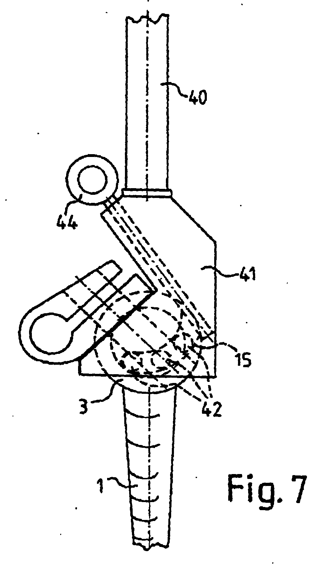

- Figur 7: eine Pedikelschraube mit einer dritten Ausführungsform eines aufgesetzten Verlängerungsstabes in einer Seitenansicht.

### Beschreibung der Ausführungsbeispiele:

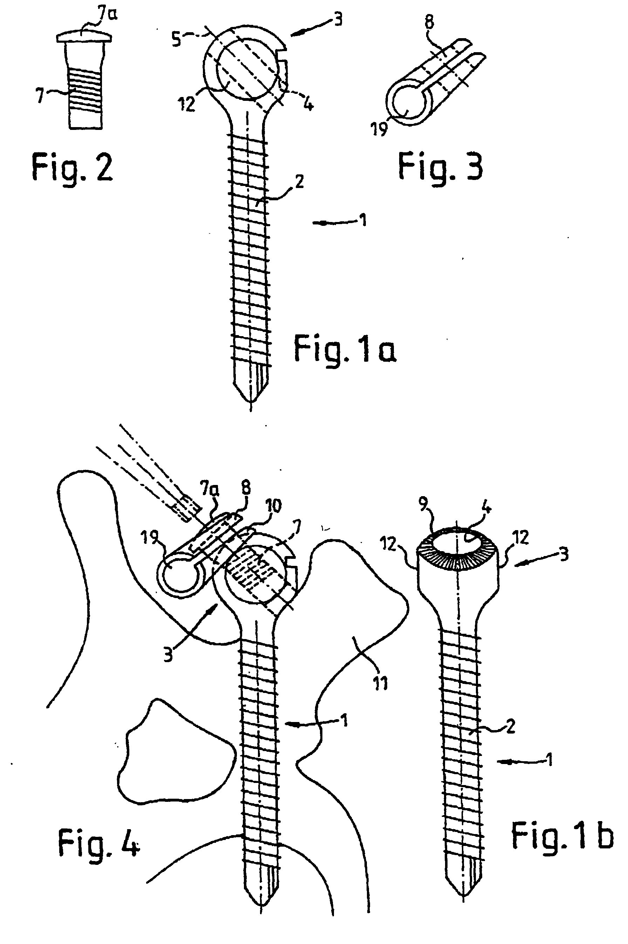

Figur 1 a und b zeigt eine Pedikelschraube 1, die einen Gewindeabschnitt 2 und einen kugelförmigen Kopf 3 aufweist. Im kugelförmigen Kopf 3 ist eine Gewindebohrung 4 vorgesehen, deren Längsachse 5 senkrecht zum eigentlichen Verlauf der kugelig gewölbten Oberfläche des Schraubenkopfes an dieser Stelle ausgerichtet ist. Beispielhaft ist in Figur 1 a die Längsachse 5 der Gewindebohrung 4 gegenüber der Längsachse 6 der Schraube um 45 Grad geneigt.

Um mehrere Pedikelschrauben 1 für die Wirbelsäulen-Stabilisierung mit einem Implantat in Form eines Längsträgers verbinden zu können, wird in der Gewindebohrung 4 im Schraubenkopf ein Gewindebolzen 7 zur Befestigung einer Klemmschelle 8 angeordnet. In der Klemmschelle 8 kann in deren kreisförmiger Öffnung 19 ein stabförmiger Längsträger (nicht dargestellt) der die Verbindung zu wenigstens einer weiteren Schraube herstellt, festgeklemmt werden. Damit die Klemmschelle 8 verdrehsicher am kugelförmigen Kopf 3 befestigt werden kann, ist im Bereich der Öffnung der Gewindebohrung 4 eine Verzahnung 9 vorhanden. Vorzugsweise weist der am kugelförmigen Kopf 3 anliegende Schenkel 10 der Klemmschelle 8 eine dazu passende Verzahnung auf.

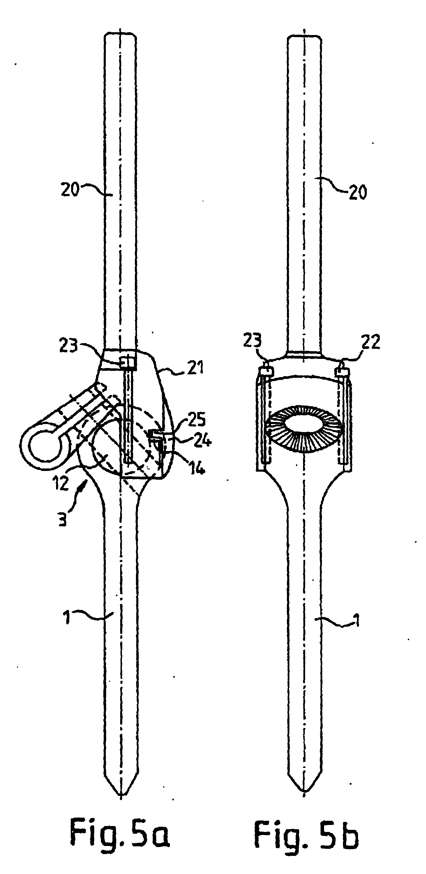

Der Gewindebolzen 7 weist einen Kopf 7 a mit z. B. einem Innensechskant auf, der sich mit einem in Figur 4 angedeuteten Schlüssel in einfacher Weise in die Gewindebohrung 4 eindrehen laßt. Figur 4 zeigt die Pedikelschraube 1 mit Klemmschelle 8 und eingedrehtem Gewindebolzen 7 in einem Wirbelkörper 11 angeordnet. Dabei ist die Pedikelschraube 1 bis zum kugelförmigen Kopf 3 eingedreht, so daß dieser sogar im unteren Bereich am Knochen anliegt. Eine solche Positionierung wird durch den kugelförmig ausgestalteten Schraubenkopf 3 ermöglicht. Denn durch diese Ausgestaltung läßt sich der Verlängerungsstab eines Manipulatorsystems am Schraubenkopf fixieren, ohne daß der Verlängerungsstab hierzu den Schraubenkopf untergreifen muß.

In den Figuren 5 a und b ist eine Pedikelschraube 1 mit einem Verlängerungsstab 20 in einer ersten Ausführungsform abgebildet. Am vorderen Ende des Verlängerungsstabes 20 ist ein Schalenelement 21 angebracht, das den kugelförmigen 3 der Petikelschraube 1 teilweise umschließt. Das Schalenelement 21 liegt an abgeflachten Abschnitten 12 des Schraubenkopfe 3 an, wodurch eine erste Verdrehsicherung geschaffen wird, Stifte 22, 23, die in entsprechenden Nuten nicht dargestellt im kugelförmigen Kopf 3 der Pedikelschraube 1 und im Schalenelement 21 verlaufen verhindern eine Bewegung des Verlängerungsstabes in eine Richtung parallel zur den abgeflachten Abschnitten 12. Um eine Bewegung des Verlängerungsstabes in Längsrichtung der Schraube zu verhindern, ist ein federnd gelagertes Rastelement 24 vorgesehen, daß mit einer Nase 25 in eine entsprechend angeordnete Nut bzw. Ausnehmung 14 am kugelförmigen Kopf 3 eingreift. Sobald das Rastelement 24 gelöst ist, läßt sich jedoch der Verlängerungsstab vom kugelförmigen Kopf 3 nach oben abziehen.

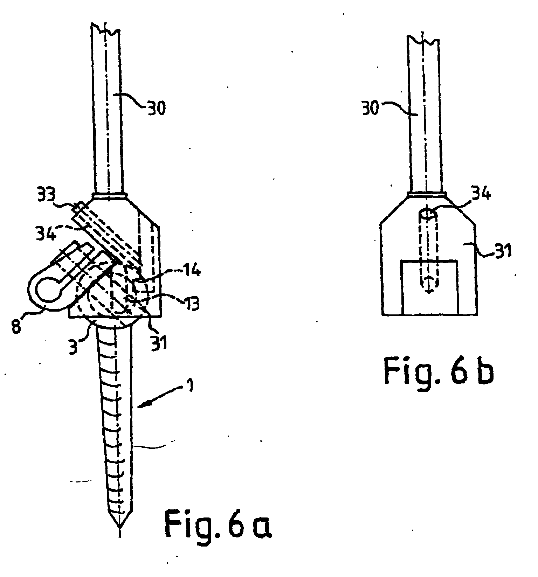

Eine weitere Ausführungsform eines Verlängerungsstabes 30 ist in den Figuren 6 a und b abgebildet. Um diesen Verlängerungsstab 30 am Kopf einer Pedikelschraube 1 anzubringen, werden gegenüberliegende Zapfen (nicht gezeigt), welche in einem Schalenelement 31 am vorderen Ende des Verlängerungsstabes 30 angeordnet sind, in eine am Kopf 3 der Pedikelschraube 1 auf beiden Seiten angeordnete Nut 13 eingeführt bis ein Klinkenelement, welches im Bereich der Rückseite des einseitig offenen Schalenelemente 31 angeordnet ist, in eine Ansnehmung 14 am kugelförmigen Kopf 3 der Pedikelschraube 1 einrastet (das Klinkenelement ist nicht dargestellt). Um das Klinkenelement zum Abziehen des Verlängerungsstabes 30 entriegeln zu können, wird ein Stift 33 in einer Bohrung 34 von vorne, das heißt von der offenen Seite des Schalenelements 31 aus gesehen, gegen das Klinkenelement gedrückt. Sofern eine Klemmschelle 8 mit Längsträger (nicht dargestellt) ein gerades Abziehen oder Anbringen verhindern, wird der Verlängerungsstab 30 nach Lösen des Klinkenelements in einer kombinierten Zug- und Schwenkbewegung entfernt.

Ein drittes Ausführungsbeispiel eines Verlängerungsstabes 40 weist zu Befestigung am kugelförmigen Kopf 3 einer Pedikelschraube 1 ein Schalenelement 41 auf, das gegenüberliegende Vorsprünge in Form von einem oder mehreren Zapfen 42, gebogenen Stegen oder dergleichen besitzt, welche in eine gebogene Nuten 15, die auf entsprechend gegenüberliegenden Seiten am kugelförmigen Kopf der Pedikelschraube 1 angeordnet sind, einschwenkt werden. Zur Blockade der Schwenkbewegung wird in eine z. B. mittig angeordnete Bohrung 43 ein Stift 44 eingeschoben. Nach Entfernen des Stifts 44 kann der Verlängerungsstab 40 mit Schalenelement 41 dann wieder durch Ausschwenken entfernt werden.

### Bezugszeichenliste:

- 1: Pedikelschraube
- 2: Gewindeabschnitt
- 3: Kopf
- 4: Gewindebohrung
- 5: Längsachse
- 6: Längsachse
- 7: Gewindebolzen
- 7 a: Kopf
- 8: Klemmschelle
- 9: Verzahnung
- 10: Schenkel
- 11: Wirbelkörper
- 12: abgeflachter Abschnitt
- 13: Nut
- 14: Ansnehmung
- 15: bogenförmige Nut
- 20: Verlängerungsstab
- 21: Schalenelement
- 22: Stift
- 23: Stift
- 24: Rastelement
- 25: Nase
- 30: Verlängerungsstab
- 31: Schalenelement
- 32: Klinkenelement
- 33: Stift
- 34: Bohrung
- 40: Verlängerungsstab
- 41: Schalenelement
- 42: Zapfen
- 43: Bohrung
- 44: Stift

## Patentansprüche

1. Schraube zur Anbringung eines Implantats für die Wirbelsäulenstabilisierung mit einem Gewindeabschnit zum Eindrehen in einen Wirbelkörper (11) und einem Schraubenkopf, in welchem eins Gewindebohrung angeordnet ist, dadurch gekennzeichnet, daß der Schraubenkopf (3) kugelförmig ist, wobei der Oberflächenbereich des Schraubenkopfs um die Gewindebohrungsöffnung ebenfalls kuglig gewölbt ist und eine Verzahnung (9) aufweist.

2. Schraube nach Anspruch 1, dadurch gekennzeichnet, daß die Längsachse der Gewindebohrung senkrecht zur kuglig gewölbten Oberfläche des Schraubenkopfs verläuft.

3. Schraube nach Anspruch 1 oder 2, dadurch gekennzeichnet, daß die Längsachse (5) der Gewindebohrung (4) zur Längsachse (6) der Schraube (1) um einen Winkel von 20 bis 50° geneigt ist.

4. Schraube nach einem der vorhergehenden Ansprüche, dadurch gekennzeichnet, daß der kugelförmige Schraubenkopf (3) zur Anbringung eines Verlängerungsstabs (20, 30, 40) zwei im Wesentlichen parallel zur Längsachse der Schraube zur Gewindebohrung symmetrisch angeordnete abgeflachte Abschnitte (12) aufweist.

5. Schraube nach Anspruch 4, dadurch gekennzeichnet, daß im Bereich der abgeflachten Abschnitte (12) eine bogenförmige Nut (15) vorgesehen ist, in welche sich Vorsprünge (42) eines Verlängerungsstabes (40) einführen lassen.

6. Schrauben nach einem der vorhergehenden Ansprüche, dadurch gekennzeichnet, daß der kugelförmige Schraubenkopf (3) zur Anbringung eines Verlängerungsstabes (29, 30, 40) auf der der Öffnung der Gewindebohrung entgegengesetzten Seite eine Ausnehmung (14) aufweist.

7. Schraube nach einem der vorhergehenden Ansprüche, dadurch gekennzeichnet, daß an der Schraube zur Befestigung eines Längsträgers für die Verbindung mehrerer Schrauben eine Klemmschelle (8) mittels eines Gewindebolzens (7) angeordnet ist, der in die Gewindebohrung (4) eingeschraubt wird.

8. Schraube nach Anspruch 7, dadurch gekennzeichnet, daß der min Kugelkopf anliegende Schenkel (10) der Klemmschelle (8) dem Kugelkopf angeformt ist und eine Verzahnung aufweist, die zu der am Kugelkopf angebrachten Verzahnung (9) paßt.

9. Verlängerungsstab zum Positionieren und Eindrehen einer Schraube zur Anbringung eines Implantats für die Wirbelsäulenstabiliesierung, insbesondere nach einem der vorhergehenden Ansprüche, welcher derart ausgestaltet ist, daß er sich am Kopf einer Schraube durch eine Schwenkbewegung verankert läßt.

10. Verlängerungsstab nach Anspruch 9, welcher an einem den Kopf einer Schraube umgreifenden Schalenelement (41) gegenüberliegende Vorsprünge (42) aufweist, die in Führungsnuten (15) am Kopf einer Schraube einschwenkbar sind.

11. Verlängerungsstab nach Anspruch 9 oder 10, dadurch gekennzeichnet, daß ein Stiftelement (44) zur Blockierung der Schwenkbewegung vorgesehen ist.

12. Verlängerungsstab zum Positionieren und Eindrehen einer Schraube zur Anbringung eines Implantats für die Wirbelsäulenstabilisierung, insbesondere nach einem der vorhergehenden Ansprüche, welcher ein Klinkenorgan (24, 32) aufweist, das in eine Ausnehmung (14) einrastbar ist, die am Kopf (3) einer Schraube vorgesehen ist.
